(19) Europäisches Patentamt
European Patent Office
Office européen des brevets

(11) EP 1 072 249 B1

(12) # EUROPÄISCHE PATENTSCHRIFT

(45) Veröffentlichungstag und Bekanntmachung des Hinweises auf die Patenterteilung:
**05.07.2006 Patentblatt 2006/27**

(51) Int Cl.:
***A61K 8/04*** *(2006.01)* ***A61K 8/36*** *(2006.01)*
***A61K 8/37*** *(2006.01)* ***A61K 8/34*** *(2006.01)*
***A61Q 19/00*** *(2006.01)*

(21) Anmeldenummer: **00114714.9**

(22) Anmeldetag: **08.07.2000**

(54) **Kosmetische und dermatologische Zubereitungen auf der Grundlage von O/W-Emulsionen**

Cosmetic and dermatological compositions based on w/o emulsions

Compositions cosmétiques et dermatologiques basées sur des émulsions huile-dans-eau

(84) Benannte Vertragsstaaten:
**AT BE CH CY DE DK ES FI FR GB GR IE IT LI LU MC NL PT SE**

(30) Priorität: **26.07.1999 DE 19934943**

(43) Veröffentlichungstag der Anmeldung:
**31.01.2001 Patentblatt 2001/05**

(73) Patentinhaber: **Beiersdorf AG**
**20253 Hamburg (DE)**

(72) Erfinder:
- **Riedel, Heidi**
**22529 Hamburg (DE)**
- **Schneider, Günther, Dr.**
**22607 Hamburg (DE)**
- **Hamer, Gunhild**
**22455 Hamburg (DE)**
- **Bleckmann, Andreas**
**22926 Ahrensburg (DE)**

(56) Entgegenhaltungen:
**FR-A- 2 732 594** **US-A- 3 448 189**
**US-A- 5 079 003**

- **PATENT ABSTRACTS OF JAPAN vol. 008, no. 126 (C-228), 13. Juni 1984 (1984-06-13) & JP 59 039815 A (SHISEIDO KK), 5. März 1984 (1984-03-05)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft kosmetische und dermatologische Emulsionen, insbesondere hautpflegende kosmetische und dermatologische Emulsionen. In einer vorteilhaften Ausführungsform betrifft die vorliegende Erfindung eine Anwendung, welche es erlaubt, die Stabilität von elektrolythaltigen Zubereitungen, insbesondere Emulsionen, bevorzugt von O/W-Emulsionen, zu steigern.

Die Haut ist das größte Organ des Menschen. Unter ihren vielen Funktionen (beispielsweise zur Wärmeregulation und als Sinnesorgan) ist die Barrierefunktion, die das Austrocknen der Haut (und damit letztlich des gesamten Organismus) verhindert, die wohl wichtigste. Gleichzeitig wirkt die Haut als Schutzeinrichtung gegen das Eindringen und die Aufnahme von außen kommender Stoffe. Bewirkt wird diese Barrierefunktion durch die Epidermis, welche als äußerste Schicht die eigentliche Schutzhülle gegenüber der Umwelt bildet. Mit etwa einem Zehntel der Gesamtdicke ist sie gleichzeitig die dünnste Schicht der Haut.

Die Epidermis ist ein stratifiziertes Gewebe, in dem die äußere Schicht, die Hornschicht (Stratum corneum), den für die Barrierefunktion bedeutenden Teil darstellt. Das heute in der Fachwelt anerkannte Hautmodell von Elias (*P. M. Elias, Structure and Function of the Stratum Corneum Permeability Barrier, Drug Dev. Res.* ***13***, *1988, 97-105*) beschreibt die Hornschicht als Zwei-Komponenten-System, ähnlich einer Ziegelsteinmauer (Ziegelstein-Mörtel-Modell). In diesem Modell entsprechen die Hornzellen (Komeozyten) den Ziegelsteinen, die komplex zusammengesetzte Lipidmembran in den Interzellularräumen entspricht dem Mörtel. Dieses System stellt im wesentlichen eine physikalische Barriere gegen hydrophile Substanzen dar, kann aber aufgrund seiner engen und mehrschichtigen Struktur gleichermaßen auch von lipophilen Substanzen nur schwer passiert werden.

Die vorliegende Erfindung betrifft in einer besonderen Ausführungsform kosmetische oder pharmazeutische Zubereitungen mit vermindertem Klebrigkeitsgefühl, Verfahren zu ihrer Herstellung sowie die Verwendung von Wirkstoffen zur Herabminderung des Klebrigkeitsgefühles kosmetischer Zubereitungen.

Außer ihrer Barrierewirkung gegen externe chemische und physikalische Einflüsse tragen die epidermalen Lipide auch zum Zusammenhalt der Hornschicht bei und haben Einfluß auf die Hautglätte. Im Gegensatz zu den Talgdrüsenlipiden, die keinen geschlossenen Film auf der Haut ausbilden, sind die epidermalen Lipide über die gesamte Hornschicht verteilt.

Das äußerst komplexe Zusammenwirken der feuchtigkeitsbindenden Substanzen und der Lipide der oberen Hautschichten ist für die Regulation der Hautfeuchte sehr wichtig. Daher enthalten Kosmetika in der Regel, neben ausgewogenen Lipidabmischungen und Wasser, wasserbindende Substanzen.

Neben der chemischen Zusammensetzung ist jedoch auch das physikalische Verhalten dieser Substanzen von Bedeutung. Daher ist die Entwicklung von sehr gut bioverträglichen Emulgatoren bzw. Tensiden wünschenswert. Damit formulierte Produkte unterstützen die flüssigkristalline Organisation der Interzellularlipide des Stratum Corneums und verbessern so die Barriereeigenschaften der Hornschicht. Besonders vorteilhaft ist es, wenn deren Molekülbestandteile aus natürlicherweise in der Epidermis vorkommenden Substanzen bestehen.

Unter kosmetischer Hautpflege ist in erster Linie zu verstehen, daß die natürliche Funktion der Haut als Barriere gegen Umwelteinflüsse (z.B. Schmutz, Chemikalien, Mikroorganismen) und gegen den Verlust von körpereigenen Stoffen (z.B. Wasser, natürliche Fette, Elektrolyte) gestärkt oder wiederhergestellt wird.

Wird diese Funktion gestört, kann es zu verstärkter Resorption toxischer oder allergener Stoffe oder zum Befall von Mikroorganismen und als Folge zu toxischen oder allergischen Hautreaktionen kommen.

Ziel der Hautpflege ist es ferner, den durch tägliche Waschen verursachten Fett- und Wasserverlust der Haut auszugleichen. Dies ist gerade dann wichtig, wenn das natürliche Regenerationsvermögen nicht ausreicht. Außerdem sollen Hautpflegeprodukte vor Umwelteinflüssen, insbesondere vor Sonne und Wind, schützen und die Hautalterung verzögern.

Medizinische topische Zusammensetzungen enthalten in der Regel ein oder mehrere Medikamente in wirksamer Konzentration. Der Einfachheit halber wird zur sauberen Unterscheidung zwischen kosmetischer und medizinischer Anwendung und entsprechenden Produkten auf die gesetzlichen Bestimmungen der Bundesrepublik Deutschland verwiesen (z. B. Kosmetikverordnung, Lebensmittel- und Arzneimittelgesetz).

Übliche kosmetische Darreichungsformen sind Emulsionen. Darunter versteht man im allgemeinen ein heterogenes System aus zwei miteinander nicht oder nur begrenzt mischbaren Flüssigkeiten, die üblicherweise als Phasen bezeichnet werden. Die eine liegt dabei in Form von Tröpfchen vor (disperse oder innere Phase), während die andere Flüssigkeit eine kontinuierliche (kohärente oder innere Phase) bildet. Seltenere Darreichungsformen sind multiple Emulsionen, also solche, welche in den Tröpfchen der dispergierten (oder diskontinuierlichen) Phase ihrerseits Tröpfchen einer weiteren dispergierten Phase enthalten, z.B. W/O/W-Emulsionen und O/W/O-Emulsionen.

Neuere Erkenntnisse führten in letzter Zeit zu einem besseren Verständnis praxisrelevanter kosmetischer Emulsionen. Dabei geht man davon aus, daß die im Überschuß eingesetzten Emulgatorgemische lamellare flüssigkristalline Phasen bzw. kristalline Gelphasen ausbilden. In der Gelnetzwerktheorie werden Stabilität und physikochemische Eigenschaften solcher Emulsionen auf die Ausbildung von viskoelastischen Gelnetzwerken zurückgeführt.

Um die Metastabilität von Emulsionen gewährleisten zu können, sind in der Regel grenzflächenaktive Substanzen, also Emulgatoren, nötig. An sich ist die Verwendung der üblichen kosmetischen Emulgatoren völlig unbedenklich. Dennoch können Emulgatoren, wie letztlich jede chemische Substanz, im Einzelfalle allergische oder auf Überempfindlichkeit des Anwenders beruhende Reaktionen hervorrufen. So ist bekannt, daß bei manchen besonders empfindlichen Personen bestimmte Lichtdermatosen durch gewisse Emulgatoren und gleichzeitige Einwirkung von Sonnenlicht ausgelöst werden.

Es ist möglich, emulgatorfreie Zubereitungen herzustellen, welche beispielsweise in einer wäßrigen Phase dispergierte Öltröpfchen, ähnlich einer O/W-Emulsion, aufweisen.

Voraussetzung dafür kann sein, daß die kontinuierliche wäßrige Phase ein die dispergierte Phase stabilisierendes Gelgerüst aufweist und andere Umstände mehr. Solche Systeme werden gelegentlich Hydrodispersionen oder Oleodispersionen genannt, je nachdem, welches die disperse und welches die kontinuierliche Phase darstellt.

Es ist für die kosmetische Galenik aber weder nötig noch möglich, auf Emulgatoren ganz zu verzichten, zumal eine gewisse Auswahl an besonders milden Emulgatoren existiert. Allerdings besteht ein Mangel des Standes der Technik an einer befriedigend großen Vielfalt solcher Emulgatoren, welche dann auch das Anwendungsspektrum entsprechend milder und hautverträglicher kosmetischer Zubereitungen deutlich verbreitern würde.

So war eine Aufgabe der vorliegenden Erfindung, kosmetische bzw. dermatologische Zubereitungen mit hervorragenden hautpflegenden Eigenschaften zur Verfügung zu stellen.

Ein Nachteil insbesondere von O/W-Emulsionen ist oft deren mangelnde Stabilität gegenüber höheren Elektrolytkonzentrationen, was sich in Phasentrennung äußert. Dies kann zwar auch bei W/O-Emulsionen gelegentlich zu Problemen führen, tritt dort aber bei weitem nicht so in den Vordergrund wie bei O/W-Systemen. Zwar läßt sich diesen oft durch geeignete Wahl des Emulgatorsystems in gewissem Maße Abhilfe schaffen, es treten dann aber ebensooft andere Nachteile auf.

Es ist andererseits oft wünschenswert, bestimmte Elektrolyte einzusetzen, um deren sonstige physikalische, chemische bzw. physiologische Eigenschaften nutzen zu können.

Üblicherweise werden die Konzentrationen aller Bestandteile einer kosmetischen oder dermatologischen Zubereitung in solchen Einheiten wie Gewichts-%, Mol-% und dergleichen angegeben. Aufgrund ihrer mehr oder minder stark ausgeprägten Dissoziation in Kationen und Anionen, oftmals in mehreren Dissoziationsstufen, erscheint es manchmal zweckmäßiger für die Schilderung der vorliegenden Erfindung und ihres technischen Hintergrundes, von der Ionenstärke eines gegebenen Elektrolytes in seiner Lösung auszugehen.

Die Ionenstärke *I* einer Elektrolytlösung ist definiert als

$$I = \frac{1}{2} \sum_i c_i \, z_i^2$$

wobei $c_i$ die Konzentrationen der einzelnen Ionensorten (in mol/l) und $z_i$ deren Ladungszahlen darstellen. Die physikalische Einheit der Ionenstärke ist die einer Konzentration (mol/l).

Eine 1-%ige ( = 0,17-molare) Kochsalzlösung hat beispielsweise eine Ionenstärke $I$ = 0,17.

Eine weitere Aufgabe der vorliegenden Erfindung war es also, Lösungswege zu kosmetischen oder dermatologischen Emulsionen, insbesondere O/W-Emulsionen, aufzudecken, welche gegenüber erhöhten Elektrolytkonzentrationen - bzw. erhöhten lonenstärken - stabil sind.

Ferner war eine Aufgabe der vorliegenden Erfindung, Zubereitungen zur Verfügung zu stellen, welche den Zustand der Haut deutlich verbessern, insbesondere die Hautrauhigkeit vermindern.

Es ist zwar bekannt, durch Hinzufügen bestimmter Substanzen, beispielsweise einiger ausgewählter Puderrohstoffe, insbesondere Talkum, ein Klebrigkeitsgefühl oder auch Schmierigkeitsgefühl zu reduzieren. Davon abgesehen, daß dieses nur selten vollständig gelingt, wird durch einen solchen Zusatz auch die Viskosität des betreffenden Produktes verändert und die Stabilität verringert.

Aufgabe war daher, all diesen den Nachteilen des Standes der Technik Abhilfe zu schaffen. Insbesondere sollten Produkte mit verringerter Klebrigkeit bzw. Schmierigkeit zur Verfügung gestellt werden. Produkte auf dem Gebiete der pflegenden Kosmetik, der dekorativen Kosmetik und der pharmakologischen Galenik sollten gleichermaßen von den geschilderten Nachteilen des Standes der Technik befreit werden.

Weiterhin war es eine Aufgabe der Erfindung, kosmetische Grundlagen für kosmetische Zubereitungen zu entwickeln, die sich durch gute Hautverträglichkeit auszeichnen.

Ferner war eine Aufgabe der vorliegenden Erfindung, Produkte mit einer möglichst breiten Anwendungsvielfalt zur Verfügung zu stellen. Beispielsweise sollten Grundlagen für Zubereitungsformen wie Reinigungsemulsionen, Gesichts- und Körperpflegezubereitungen, aber auch ausgesprochen medizinisch-pharmazeutische Darreichungsformen geschaffen werden, zum Beispiel Zubereitungen gegen Akne und andere Hauterscheinungen.

Es hat sich überraschend gezeigt, und darin liegt die Lösung dieser Aufgaben, daß kosmetische und dermatologische Zubereitungen in Form von O/W-Emulsionen, enthaltend

(1) 0,1 bis zu 5 Gew.-%, bezogen auf das Gesamtgewicht der Zubereitungen, einer oder mehrerer $C_{14}$-$C_{22}$-Fettsäuren,
(2) 0,2 bis zu 10 Gew.-%, bezogen auf das Gesamtgewicht der Zubereitungen, eines oder mehrerer Mono- und/oder Diglyceride von Fettsäuren,
(3) 0,1 bis zu 5 Gew.-%, bezogen auf das Gesamtgewicht der Zubereitungen, eines oder mehrerer ethoxylierter Fettsäureester,
wobei die Summe aus (1), (2) und (3) maximal 12 Gew. % beträgt,
(4) 0,5 bis 10 Gew.-%, bezogen auf das Gesamtgewicht der Zubereitungen, eines oder mehrerer unpolarer Lipide,
(5) 0,5 - 7,5 Gew.-%, bezogen auf das Gesamtgewicht der Zubereitungen, eines oder mehrerer Fettalkohole
(6) 0,5 - 7,5 Gew.-%, bezogen auf das Gesamtgewicht der Zubereitungen, eines oder mehrerer lipophiler Konsistenzgeber eines Schmelz- oder Tropfpunktes ≥ 30 °C
(7) 0,5 - 10 Gew.-%, bezogen auf das Gesamtgewicht der Zubereitungen, an einem oder mehreren Siliconölen,
(8) wobei die gesamte Lipidphase bis zu 40 Gew.-% polare Lipide enthalten kann, bezogen auf das Gesamtgewicht der Lipidphase,

den Nachteilen des Standes der Technik abhelfen.

Es war für den Fachmann daher nicht vorauszusehen gewesen, daß die erfindungsgemäßen Zubereitungen

- besser als feuchtigkeitsspendende Zubereitungen wirken,
- besser die Hautglättung fördern,
- sich durch bessere Pflegewirkung auszeichnen,
- besser als Vehikel für kosmetische und medizinisch-dermatologische Wirkstoffe dienen
- höhere Stabilität gegenüber Zerfall in Öl- und Wasserphasen aufweisen und
- sich durch bessere Bioverträglichkeit auszeichnen würden
- sich durch besseres Hautgefühl und durch höhere kosmetische Eleganz auszeichnen würden

als die Zubereitungen des Standes der Technik.

Die erfindungsgemäßen Zubereitungen sind sowohl fließfähig als auch crèmeartig formulierbar, besitzen sehr gute kosmetische Eigenschaften, insbesondere was die Klebrigkeit betrifft, und weisen eine sehr gute Hautverträglichkeit sowie Hautpflegeleistung auf.

Vorteilhafte Ausführungsformen der vorliegenden Erfindung betreffen kosmetische und dermatologische Zubereitungen gemäß Hauptanspruch, enthaltend

(1) 0,5 bis zu 1,0 Gew.-%, bezogen auf das Gesamtgewicht der Zubereitungen, einer oder mehrerer $C_{14}$-$C_{22}$-Fettsäuren.
Vorteilhafte Ausführungsformen der vorliegenden Erfindung betreffen auch kosmetische und dermatologische Zubereitungen gemäß Hauptanspruch, enthaltend
(2) 2,5 bis zu 3,0 Gew.-%, bezogen auf das Gesamtgewicht der Zubereitungen, eines oder mehrerer Mono- und/oder Diglyceride von Fettsäuren.
Vorteilhafte Ausführungsformen der vorliegenden Erfindung betreffen auch kosmetische und dermatologische Zubereitungen gemäß Hauptanspruch, enthaltend
(3) 1,0 bis zu 1,5 Gew.-%, bezogen auf das Gesamtgewicht der Zubereitungen, eines oder mehrerer ethoxylierter Fettsäureester.

Insbesondere vorteilhafte Ausführungsformen der vorliegenden Erfindung werden erhalten, wenn diese kosmetische und dermatologische Zubereitungen gemäß Hauptanspruch betreffen, enthaltend

(2) bis zu 10 Gew.-%, bezogen auf das Gesamtgewicht der Zubereitungen, an Glycerylstearat.
Insbesondere vorteilhafte Ausführungsformen der vorliegenden Erfindung werden erhalten, wenn sie kosmetische und dermatologische Zubereitungen gemäß Hauptanspruch betreffen, enthaltend
(3) bis zu 5 Gew.-%, bezogen auf das Gesamtgewicht der Zubereitungen, eines oder mehrerer ethoxylierter Fettsäureester gewählt aus der Gruppe der PEG-20- bis PEG-100-Stearate.

Es ist insbesondere vorteilhaft, Gewichtsverhältnisse zwischen den unter (1), (2) und (3) genannten Bestandteile von ungefähr 1:4:2 zu wählen.

Weiterhin werden vorteilhafte Ausführungen der vorliegenden Erfindung erhalten, wenn diese kosmetische und dermatologische Zubereitungen gemäß Hauptanspruch betreffen, enthaltend

(4) 0,5 bis 10 Gew.-%, bezogen auf das Gesamtgewicht der Zubereitungen, eines oder mehrerer unpolarer Lipide, gewählt aus der Gruppe Mineralöl und/oder Mineralwachse (u.a. Vaseline)

Weiterhin werden vorteilhafte Ausführungen der vorliegenden Erfindung erhalten, wenn diese kosmetische und dermatologische Zubereitungen gemäß Hauptanspruch betreffen, enthaltend

(5) 2,0 - 3,0 Gew.-%, bezogen auf das Gesamtgewicht der Zubereitungen, eines oder mehrerer Fettalkohole.

Weiterhin werden vorteilhafte Ausführungen der vorliegenden Erfindung erhalten, wenn diese kosmetische und dermatologische Zubereitungen gemäß Hauptanspruch betreffen, enthaltend

(6) 1,5 - 2,5 Gew.-%, bezogen auf das Gesamtgewicht der Zubereitungen, eines oder mehrerer lipophiler Konsistenzgeber eines Schmelz- oder Tropfpunktes ≥ 30 °C

Weiterhin werden vorteilhafte Ausführungen der vorliegenden Erfindung erhalten, wenn diese kosmetische und dermatologische Zubereitungen gemäß Hauptanspruch betreffen, enthaltend

(7) 5 - 8 Gew.-%, bezogen auf das Gesamtgewicht der Zubereitungen, an einem oder mehreren Siliconölen, besonders bevorzugt Cylomethicone

Im Rahmen der vorliegenden Offenbarung wird als Oberbegriff für Fette, Öle, Wachse und dergleichen gelegentlich der Ausdruck "Lipide" verwendet, wie dem Fachmanne durchaus geläufig ist. Auch werden die Begriffe "Ölphase" und "Lipidphase" synonym angewandt.

Öle und Fette unterscheiden sich unter anderem in ihrer Polarität, welche schwierig zu definieren ist. Es wurde bereits vorgeschlagen, die Grenzflächenspannung gegenüber Wasser als Maß für den Polaritätsindex eines Öls bzw. einer Ölphase anzunehmen. Dabei gilt, daß die Polarität der betreffenden Ölphase um so größer ist, je niedriger die Grenzflächenspannung zwischen dieser Ölphase und Wasser ist. Erfindungsgemäß wird die Grenzflächenspannung als ein mögliches Maß für die Polarität einer gegebenen Ölkomponente angesehen.

Die Grenzflächenspannung ist diejenige Kraft, die an einer gedachten, in der Grenzfläche zwischen zwei Phasen befindlichen Linie der Länge von einem Meter wirkt. Die physikalische Einheit für diese Grenzflächenspannung errechnet sich klassisch nach der Beziehung Kraft/Länge und wird gewöhnlich in mN/m (Millinewton geteilt durch Meter) wiedergegeben. Sie hat positives Vorzeichen, wenn sie das Bestreben hat, die Grenzfläche zu verkleinern. Im umgekehrten Falle hat sie negatives Vorzeichen. Als polar im Sinne der vorliegenden Erfindung werden Lipide angesehen, deren Grenzflächenspannung gegen Wasser weniger als 30 mN/m beträgt.

Polare Öle, sind beispielsweise solche aus der Gruppe der Lecithine und der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, wie z. B. Olivenöl, Sonnenblumenöl, Sojaöl, Erdnußöl, Rapsöl, Mandelöl, Palmöl, Kokosöl, Rizinusöl, Weizenkeimöl, Traubenkernöl, Distelöl, Nachtkerzenöl, Macadamianußöl und dergleichen mehr.

Weitere polare Ölkomponenten können gewählt werden aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, wie z.B. Jojobaöl.

Ferner können die Bestandteile der Ölphase vorteilhaft gewählt werden aus der Gruppe der Dialkylether, der Gruppe der gesättigten oder ungesättigten, verzweigten oder unverzweigten Alkohole.

Auch beliebige Abmischungen solcher Öl- und Wachskomponenten sind vorteilhaft im Sinne der vorliegenden Erfindung einzusetzen.

Unpolare Öle sind beispielsweise solche, welche gewählt werden aus der Gruppe der verzweigten und unverzweigten Kohlenwasserstoffe und -wachse, insbesondere Vaseline (Petrolatum), Paraffinöl, Squalan und Squalen, Polyolefine und hydrogenierte Polyisobutene. Unter den Polyolefinen sind Polydecene die bevorzugten Substanzen. Die nachfolgende Tabelle 1 führt Lipide auf, die als Einzelsubstanzen oder auch im Gemisch untereinander erfindungsgemäß vorteilhaft sind. Die betreffenden Grenzflächenspannungen gegen Wasser sind in der letzten Spalte angegeben. Es ist jedoch auch vorteilhaft, Gemische aus höher- und niederpolaren und dergleichen zu verwenden, sofern gewährleistet ist, daß die Gesamtpolarität der Ölphase im beanspruchten Bereich liegt.

**Tabelle 1**

| Handelsname | INCI-Bezeichnung | (mN/m) |
|---|---|---|
| Isofol® 14 T | Butyl Decanol + Hexyl Decanol + Hexyl Octanol + Butyl Octanol | 27,6 |
| Isofol® 16 | Hexyl Decanol | 24,3 |
| Eutanol® G | Octyldodecanol | 24,8 |
| Cetiol® OE | Dicaprylyl Ether | 22,1 |
| Miglyol® 812 | Caprylic/Capric Triglyceride | 21,3 |
| Cegesoft® C24 | Octyl Palmitate | 23,1 |
| Isopropylstearat | Isopropyl Stearate | 21,9 |
| Estol® 1540 EHC | Octyl Octanoate | 30,0 |
| Finsolv® TN | $C_{12-15}$ Alkyl Benzoate | 21,8 |
| Cetiol® SN | Cetearyl Isononanoate | 28,6 |
| Dermofeel® BGC | Butylene Glycol Caprylate/Caprate | 21,5 |
| Trivent® OCG | Tricaprylin | 20,2 |
| MOD | Octyldodecyl Myristate | 22,1 |
| Cosmacol® ETI | Di-$C_{12-13}$Alkyl Tartrate | 29,4 |
| Miglyol® 829 | Caprylic/Capric Diglyceryl Succinate | 29,5 |
| Prisorine® 2036 | Octyl Isostearate | 29,7 |
| Tegosoft® SH | Stearyl Heptanoate | 28,7 |
| Abil® Wax 9840 | Cetyl Dimethicone | 25,1 |
| Cetiol® LC | Coco-Caprylate/Caprate | 24,8 |
| IPP | Isopropyl Palmitate | 22,5 |
| Luvitol® EHO | Cetearyl Octanoate | 28,6 |
| Cetiol® 868 | Octyl Stearate | 28,4 |

Es wird erfindungsgemäß bevorzugt, das oder die Siliconöle der erfindungsgemäßen Zubereitungen aus der Gruppe der cyclischen und/oder linearen Silicone zu wählen, welche im Rahmen der vorliegenden Offenbarung auch als "Siliconöle" bezeichnet werden. Solche Silicone oder Siliconöle können als Monomere vorliegen, welche in der Regel durch Strukturelemente charakterisiert sind, wie folgt:

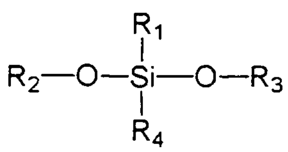

Als erfindungsgemäß vorteilhaft einzusetzende lineare Silicone mit mehreren Siloxyleinheiten werden im allgemeinen durch Strukturelemente charakterisiert wie folgt:

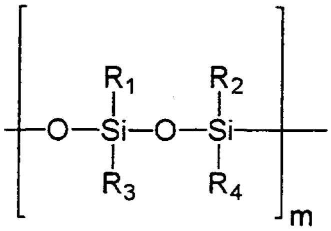

,

wobei die Siliciumatome mit gleichen oder unterschiedlichen Alkylresten und/oder Arylresten substituiert werden können, welche hier verallgemeinernd durch die Reste $R_1$ - $R_4$ dargestellt sind (will sagen, daß die Anzahl der unterschiedlichen Reste nicht notwendig auf bis zu 4 beschränkt ist). m kann dabei Werte von 2 - 200.000 annehmen.

Erfindungsgemäß vorteilhaft einzusetzende cyclische Silicone werden im allgemeinen durch Strukturelemente charakterisiert, wie folgt

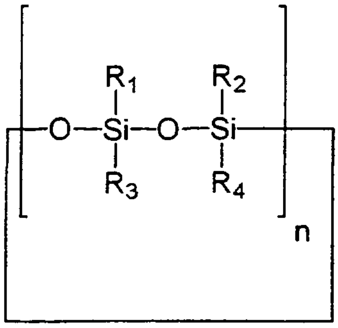

wobei die Siliciumatome mit gleichen oder unterschiedlichen Alkylresten und/oder Arylresten substituiert werden können, welche hier verallgemeinernd durch die Reste $R_1$ - $R_4$ dargestellt sind (will sagen, daß die Anzahl der unterschiedlichen Reste nicht notwendig auf bis zu 4 beschränkt ist). n kann dabei Werte von 3/2 bis 20 annehmen. Gebrochene Werte für n berücksichtigen, daß ungeradzahlige Anzahlen von Siloxylgruppen im Cyclus vorhanden sein können.

Vorteilhaft wird Phenyltrimethicon als Siliconöl gewählt. Auch andere Silikonöle, beispielsweise Dimethicon, Phenyldimethicon, Cyclomethicon (Octamethylcyclotetrasiloxan) beispielsweise Hexamethylcyclotrisiloxan, Polydimethylsiloxan, Poly(methylphenylsiloxan), Cetyldimethicon, Behenoxydimethicon sind vorteilhaft im Sinne der vorliegenden Erfindung zu verwenden.

Vorteilhaft sind ferner Mischungen aus Cyclomethicon und Isotridecylisononanoat, sowie solche aus Cyclomethicon und 2-Ethylhexylisostearat.

Es ist aber auch vorteilhaft, Silikonöle ähnlicher Konstitution wie der vorstehend bezeichneten Verbindungen zu wählen, deren organische Seitenketten derivatisiert, beispielsweise polyethoxyliert und/oder polypropoxyliert sind. Dazu zählen beispielsweise Polysiloxan-polyalkyl-polyether-copolymere wie das Cetyl-Dimethicon-Copolyol, das (Cetyl-Dimethicon-Copolyol (und) Polyglyceryl-4-Isostearat (und) Hexyllaurat)

Besonders vorteilhaft können das oder die Siliconöle auch gewählt werden aus der Gruppe der Silicone der allgemeinen Struktur

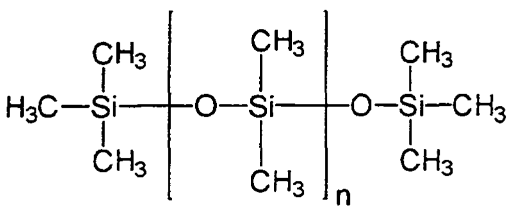

,

bezogen auf das Gesamtgewicht der Zubereitung, aufweisen, wobei n Zahlen zwischen 5 und 50 annehmen kann. Es ist dabei von Vorteil, den Durchschnittswert n in aus dem Bereich von 12 -16 zu wählen. Als ein besonders vorteilhafter Vertreter solcher Siliconöle hat sich das Siliconöl AK 10 der Gesellschaft Wacker-Chemie GmbH herausgestellt, dessen

Durchschnittswert für n bei etwa 14 liegt.

Erfindungsgemäß bevorzugte Zubereitungen enthalten vorteilhaft einen Anteil von mindestens 5 Gew.-%, bevorzugt mindestens 10 Gew.-%, an einem oder mehreren Siliconölen.

Als Grundbestandteile der erfindungsgemäßen Zubereitungen können verwendet werden:

- Wasser oder wäßrige Lösungen
- wäßrige ethanolische Lösungen
- natürliche Öle und/oder chemisch modifizierte natürliche Öle und/oder synthetische Öle;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder - monobutylether, Diethylenglykolmonomethyl- oder -monoethylether und analoge Produkte.

Insbesondere werden Gemische der vorstehend genannten Lösungsmittel verwendet.

Die Ölphase der Emulsionen im Sinne der vorliegenden Erfindung besteht erfindungsgemäß vorzugsweise vorwiegend aus Komponenten der unter Punkt (4) aufgeführten Art, wobei es allerdings möglich ist, ohne große Nachteile in Kauf zu nehmen, bis zu 50 Gew.-%, vorzugsweise bis zu 40 Gew.-% des Gesamtgewichtes der Ölkomponenten aus der Gruppe anderer Ölkomponenten zu wählen. Diese können dann vorteilhaft gewählt werden aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Ferner kann die Ölphase vorteilhaft gewählt werden aus der Gruppe der verzweigten und unverzweigten Kohlenwasserstoffe und -wachse, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnußöl, Rapsöl, Mandelöl, Palmöl, Kokosöl, Palmkernöl und dergleichen mehr, sofern die im Hauptanspruch geforderten Bedingungen eingehalten werden.

Erfindungsgemäß vorteilhaft zu verwendende Fett- und/oder Wachskomponenten können aus der Gruppe der pflanzlichen Wachse, tierischen Wachse, Mineralwachse und petrochemischen Wachse gewählt werden. Erfindungsgemäß günstig sind beispielsweise Candelillawachs, Carnaubawachs, Japanwachs, Espartograswachs, Korkwachs, Guarumawachs, Reiskeimölwachs, Zuckerrohrwachs, Beerenwachs, Ouricurywachs, Montanwachs, Jojobawachs, Shea Butter, Bienenwachs, Schellackwachs, Walrat, Lanolin (Wollwachs), Bürzelfett, Ceresin, Ozokerit (Erdwachs), Paraffinwachse und Mikrowachse, sofern die im Hauptanspruch geforderten Bedingungen eingehalten werden.

Weitere vorteilhafte Fett- und/oder Wachskomponenten sind chemisch modifizierte Wachse und synthetische Wachse, wie beispielsweise die unter den Handelsbezeichnungen Syncrowax HRC (Glyceryltribehenat), Syncrowax HGLC ($C_{16-36}$ -Fettsäuretriglycerid) und Syncrowax AW 1C ($C_{18-36}$ -Fettsäure) bei der CRODA GmbH erhältlichen sowie Montanesterwachse, Sasolwachse, hydrierte Jojobawachse, synthetische oder modifizierte Bienenwachse (z. B. Dimethicon Copolyol Bienenwachs und/oder $C_{30-50}$ -Alkyl Bienenwachs), Polyalkylenwachse, Polyethylenglykolwachse, aber auch chemisch modifizierte Fette, wie z. B. hydrierte Pflanzenöle (beispielsweise hydriertes Ricinusöl und/oder hydrierte Cocosfettglyceride), Triglyceride, wie beispielsweise Trihydroxystearin, Fettsäuren, Fettsäureester und Glykolester, wie beispielsweise $C_{20-40}$-Alkylstearat, $C_{20-40}$-Alkylhydroxystearoylstearat und/oder Glykolmontanat. Weiter vorteilhaft sind auch bestimmte Organosiliciumverbindungen, die ähnliche physikalische Eigenschaften aufweisen wie die genannten Fett- und/oder Wachskomponenten, wie beispielsweise Stearoxytrimethylsilan sofern die im Hauptanspruch geforderten Bedingungen eingehalten werden.

Erfindungsgemäß können die Fett- und/oder Wachskomponenten sowohl einzeln als auch im Gemisch vorliegen.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen sofern die im Hauptanspruch geforderten Bedingungen eingehalten werden.

Vorteilhaft wird die Ölphase gewählt aus der Gruppe 2-Ethylhexylisostearat, Octyldodecanol, Isotridecylisono-

nanoat, Isoeicosan, 2-Ethylhexylcocoat, $C_{12-15}$-Alkylbenzoat, Capryl-Caprinsäure-triglycerid, Dicaprylylether sofern die im Hauptanspruch geforderten Bedingungen eingehalten werden.

Besonders vorteilhaft sind Mischungen aus $C_{12-15}$-Alkybenzoat und 2-Ethylhexylisostearat, Mischungen aus $C_{12-15}$-Alkybenzoat und Isotridecylisononanoat sowie Mischungen aus $C_{12-15}$-Alkybenzoat, 2-Ethylhexylisostearat und Isotridecylisononanoat, sofern die im Hauptanspruch geforderten Bedingungen eingehalten werden.

Erfindungsgemäße O/W-Emulsionen können vorteilhaft mit Hilfe der üblichen O/W-Emulgatoren, gewünschtenfalls unter Zuhilfenahme von W/O-Emulgatoren bzw. weiteren Coemulgatoren hergestellt werden.

O/W-Emulsionen entsprechend der vorliegenden Erfindung enthalten einen oder mehrere Emulgatoren, gewünschtenfalls vorteilhaft gewählt aus der Gruppe der folgenden Substanzen, die in der Regel als W/O-Emulgatoren wirken:

Sorbitanstearat, Sorbitanoleat,, Lecithin, Glyceryllanolat, Lanolin, mikrokristallines Wachs (Cera microcristallina) im Gemisch mit Paraffinöl (Paraffinum liquidum), Ozokerit, hydriertem Ricinusöl, Glycerylisostearat, Polyglyceryl-3-Oleat, Wollwachssäuregemische, Wollwachsalkoholgemische, Pentaerythrithylisostearat, Polyglyceryl-3 Diisostearat, Sorbitan Oleat im Gemisch mit hydriertem Ricinusöl, Bienenwachs (Cera alba) und Stearinsäure, Natriumdihydroxycetylphosphat im Gemisch mit Isopropylhydroxycetylether, Methylglucosedioleat, Methylglucosedioleat im Gemisch mit Hydroxystearat und Bienenwachs, Mineralöl im Gemisch mit Petrolatum und Ozokerit und Glyceryloleat und Lanolinalkohol, Petrolatum im Gemisch mit Ozokerit und hydriertem Ricinusöl und Glycerylisostearat und Polyglyceryl-3-oleat, PEG-7-hydriertes Ricinusöl, Sorbitanoleat im Gemisch mit PEG-2-hydriertem Ricinusöl, Ozokerit und hydriertem Ricinusöl, Sorbitanisostearat im Gemisch mit PEG-2-hydriertem Ricinusöl, Polyglyceryl-4-isostearat, Polyglyceryl-4-isostearat im Gemisch mit Cetyldimethiconcopolyol und Hexyllaurat, Laurylmethiconcopolyol, Cetyldimethiconcopolyol, Acrylat/ $C_{10-30}$-Alkylacrylat-Crosspolymer, Sorbitanisostearat, Poloxamer 101, Polyglyceryl-2-dipolyhydroxystearat, Polyglyceryl-3-Diisostearat, Polyglyceryl-4-dipolyhydroxystearat, PEG-30-dipolyhydroxystearat, Diisostearoylpolyglyceryl-3-diisostearat, Polyglyceryl-2-dipolyhydroxystearat, Polyglyceryl-3-dipolyhydroxystearat, Polyglyceryl-4-dipolyhydroxystearat, Polyglyceryl-3-dioleat.

O/W-Emulsionen entsprechend der vorliegenden Erfindung enthalten gewünschtenfalls einen oder mehrere Emulgatoren, insbesondere vorteilhaft gewählt aus der Gruppe der folgenden Substanzen, die in der Regel als O/W-Emulgatoren wirken:

Glycerylstearat im Gemisch mit Ceteareth-20, Ceteareth-25, Ceteareth-6 im Gemisch mit Stearylalkohol, Cetylstearylalkohol im Gemisch mit PEG-40-Ricinusöl und Natriumcetylstearylsulfat, Triceteareth-4 Phosphat, Glycerylstearat, Natriumcetylstearylsulfat, Lecithin Trilaureth-4 Phosphat, Laureth-4 Phosphat, Stearinsäure, Propylenglycolstearat SE, PEG-25-hydriertes Ricinusöl, PEG-54-hydriertes Ricinusöl, PEG-6 Caprylsäure/Caprinsäureglyceride, Glyceryloleat im Gemisch mit Propylenglycol, PEG-9-Stearat, Ceteth-2, Ceteth-20, Polysorbat 60, Glycerylstearat im Gemisch mit PEG-100 Stearat, Glycerylmyristat, Glyceryllaurat, PEG-40-Sorbitanperoleat, Laureth-4, Ceteareth-3, Isostearylglycerylether, Cetylstearylalkohol im Gemisch mit Natrium Cetylstearylsulfat, Laureth-23, Steareth-2, Glycerylstearat im Gemisch mit PEG-30 Stearat, PEG-40-Stearat, Glycol Distearat, PEG-22-Dodecyl Glycol Copolymer, Polyglyceryl-2-PEG-4-Stearat, Ceteareth-20, Methylglucosesesquistearat, Steareth- 10, PEG-20-Stearat, Steareth-2 im Gemisch mit PEG-8 Distearat, Steareth-21, Steareth-20, Isosteareth-20, PEG-45/Dodecylglycol-Copolymer, Methoxy-PEG-22/Dodecylglycol-Copolymer, PEG-40-Sorbitanperoleat, PEG-40-Sorbitanperisostearat, PEG-20-Glycerylstearat, PEG-20-Glycerylstearat, PEG-8-Bienenwachs, Polyglyceryl-2-laurat, Isostearyldiglycerylsuccinat, Stearamidopropyl-PG-dimoniumchloridphosphat, Glycerylstearat SE, Ceteth-20, Triethylcitrat, PEG-20-Methylglucosesesquistearat, Ceteareth-12, Glycerylstearatcitrat, Cetylphosphat, Sorbitansesquioleat, Triceteareth-4-Phosphat, Trilaureth-4-Phosphat, Polyglycerylmethylglucosedistearat, Kaliumcetylphosphat, Isosteareth-10, Polyglyceryl-2-sesquiisostearat, Ceteth-10, Oleth-20, Isoceteth-20, Glycerylstearat im Gemisch mit Ceteareth-20, Ceteareth-12, Cetylstearylalkohol und Cetylpalmitat, Cetylstearylalkohol im Gemisch mit PEG-20 Stearat, PEG-30-Stearat, PEG-40-Stearat, PEG-100-Stearat.

Erfindungsgemäße Emulsionen im Sinne der vorliegenden Erfindung, z. B. in Form einer Hautschutzcrème, einer Hautlotion, einer kosmetischen Milch, beispielsweise in Form einer Sonnenschutzcreme oder einer Sonnenschutzmilch, sind vorteilhaft und enthalten z. B. Fette, Öle, Wachse und/oder andere Fettkörper, sowie Wasser und einen oder mehrere Emulgatoren, wie sie üblicherweise für einen solchen Typ der Formulierung verwendet werden.

Es ist dem Fachmanne natürlich bekannt, daß anspruchsvolle kosmetische Zusammensetzungen zumeist nicht ohne die üblichen Hilfs- und Zusatzstoffe denkbar sind. Darunter zählen beispielsweise Konsistenzgeber, Füllstoffe, Parfum, Farbstoffe, Emulgatoren, zusätzliche Wirkstoffe wie Vitamine oder Proteine, Lichtschutzmittel, Stabilisatoren, Insektenrepellentien, Alkohol, Wasser, Salze, antimikrobiell, proteolytisch oder keratolytisch wirksame Substanzen usw.

Mutatis mutandis gelten entsprechende Anforderungen an die Formulierung medizinischer Zubereitungen.

Medizinische topische Zusammensetzungen im Sinne der vorliegenden Erfindung enthalten in der Regel ein oder mehrere Medikamente in wirksamer Konzentration. Der Einfachheit halber wird zur sauberen Unterscheidung zwischen kosmetischer und medizinischer Anwendung und entsprechenden Produkten auf die gesetzlichen Bestimmungen der Bundesrepublik Deutschland verwiesen (z.B. Kosmetikverordnung, Lebensmittel- und Arzneimittelgesetz).

Entsprechend können kosmetische oder topische dermatologische Zusammensetzungen im Sinne der vorliegenden Erfindung, je nach ihrem Aufbau, beispielsweise verwendet werden als Hautschutzcrème, Reinigungsmilch, Sonnenschutzlotion, Nährcrème, Tages- oder Nachtcrème usw. Es ist gegebenenfalls möglich und vorteilhaft, die erfindungsgemäßen Zusammensetzungen als Grundlage für pharmazeutische Formulierungen zu verwenden.

Es ist ebenfalls von Vorteil, von den erfindungsgemäßen Eigenschaften in Form von dekorativen Kosmetika (Make-Up-Formulierungen) Gebrauch zu machen.

Günstig sind auch solche kosmetischen und dermatologischen Zubereitungen, die in der Form eines Sonnenschutzmittels vorliegen. Vorzugsweise enthalten diese neben dem erfindungsgemäß verwendeten Wirkstoff zusätzlich mindestens eine UVA-Filtersubstanz und/oder mindestens eine UVB-Filtersubstanz und/oder mindestens ein anorganisches Pigment.

Es ist aber auch vorteilhaft im Sinne der vorliegenden Erfindungen, solche kosmetischen und dermatologischen Zubereitungen zu erstellen, deren hauptsächlicher Zweck nicht der Schutz vor Sonnenlicht ist, die aber dennoch einen Gehalt an UV-Schutzsubstanzen enthalten. So werden beispielsweise in Tagescrèmes gewöhnlich UV-A- bzw. UV-B-Filtersubstanzen eingearbeitet.

Vorteilhaft können erfindungsgemäße Zubereitungen Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1 bis 6 Gew.-% beträgt, bezogen auf das Gesamtgewicht der Zubereitungen.

Die UVB-Filter können öllöslich oder wasserlöslich sein. Als öllösliche Substanzen sind z. B. zu nennen:

- 3-Benzylidencampher und dessen Derivate, z. B. 3-(4-Methylbenzyliden)campher,
- 4-Aminobenzoësäure-Derivate, vorzugsweise 4-(Dimethylamino)-benzoësäure(2-ethylhexyl)ester, 4-(Dimethylamino)benzoësäureamylester;
- Ester der Zimtsäure, vorzugsweise 4-Methoxyzimtsäure(2-ethylhexyl)ester, 4-Methoxyzimtsäureisopentylester;
- Ester der Salicylsäure, vorzugsweise Salicylsäure(2-ethylhexyl)ester, Salicylsäure (4-isopropylbenzyl)ester, Salicylsäurehomomenthylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzalmalonsäuredi(2-ethylhexyl)ester;
- 2,4,6-Trianilino-(p-carbo-2'-ethyl-1'-hexyloxy)-1,3,5-triazin

Als wasserlösliche Substanzen sind vorteilhaft:

- 2-Phenylbenzimidazol-5-sulfonsäure und deren Salze, z. B. Natrium-, Kalium-oder Triethanolammonium-Salze,
- Sulfonsäure-Derivate von Benzophenonen, vorzugsweise 2-Hydroxy-4-methoxybenzophenon-5-sulfonsäure und ihre Salze;
- Sulfonsäure-Derivate des 3-Benzylidencamphers, wie z. B. 4-(2-Oxo-3-bornylidenmethyl)benzolsulfonsäure, 2-Methyl-5-(2-oxo-3-bornylidenmethyl)sulfonsäure und ihre Salze.

Die Liste der genannten UVB-Filter, die erfindungsgemäß Verwendung finden können, soll selbstverständlich nicht limitierend sein.

Es kann auch von Vorteil sein, in erfindungsgemäßen Zubereitungen UVA-Filter einzusetzen, die üblicherweise in kosmetischen und/oder dermatologischen Zubereitungen enthalten sind. Bei solchen Filtersubstanzen handelt es sich vorzugsweise um Derivate des Dibenzoylmethans, insbesondere um 1-(4'-tert.Butylphenyl)-3-(4'-methoxyphenyl)-propan-1,3-dion und um 1-Phenyl-3-(4'-isopropylphenyl)propan-1,3-dion. Auch Zubereitungen, die diese Kombinationen enthalten, sind Gegenstand der Erfindung. Es können die gleichen Mengen an UVA-Filtersubstanzen verwendet werden, welche für UVB-Filtersubstanzen genannt wurden.

Kosmetische und/oder dermatologische Zubereitungen im Sinne der vorliegenden Erfindung können auch anorganische Pigmente enthalten, die üblicherweise in der Kosmetik zum Schutze der Haut vor UV-Strahlen verwendet werden. Dabei handelt es sich um Oxide des Titans, Zinks, Eisens, Zirkoniums, Siliciums, Mangans, Aluminiums, Cers und Mischungen davon, sowie Abwandlungen, bei denen die Oxide die aktiven Agentien sind. Besonders bevorzugt handelt es sich um Pigmente auf der Basis von Titandioxid. Es können die für die vorstehenden Kombinationen genannten Mengen verwendet werden.

Die erfindungsgemäßen kosmetischen und dermatologischen Zubereitungen können kosmetische Wirk-, Hilfs- und/oder Zusatzstoffe enthalten, wie sie üblicherweise in solchen Zubereitungen verwendet werden, z.B. Antioxidationsmittel, Konservierungsmittel, Bakterizide, Parfüme, Substanzen zum Verhindern des Schäumens, Farbstoffe, Pigmente, die färbende Wirkung haben, Verdickungsmittel, oberflächenaktive Substanzen, Emulgatoren, weichmachende, anfeuchtende und/oder feuchthaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Es ist vorteilhaft, den Zubereitungen im Sinne der vorliegenden Erfindung weitere antür ritative oder antientzündliche Wirkstoffe zuzugeben, insbesondere Batylalkohol ($\alpha$-Octadecylglycerylether), Selachylalkohol ($\alpha$-9-Octadecenylglycerylether), Chimylalkohol ($\alpha$-Hexadecylglycerylether), Bisabolol und/oder Panthenol.

Es ist ebenfalls vorteilhaft, den Zubereitungen im Sinne der vorliegenden Erfindung übliche Antioxidantien zuzufügen. Erfindungsgemäß können als günstige Antioxidantien alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z. B. Glycin, Histidin, Tyrosin, Tryptophan) und deren Derivate, Imidazole (z. B. Urocaninsäure) und deren Derivate, Peptide wie D,L-Camosin, D-Carnosin, L-Carnosin und deren Derivate (z. B. Anserin), Carotinoide, Carotine (z. B. $\alpha$-Carotin, $\beta$-Carotin, $\psi$-Lycopin) und deren Derivate, Chlorogensäure und deren Derivate, Liponsäure und deren Derivate (z.B. Dihydroliponsäure), Aurothioglucose, Propylthiouracil und andere Thiole (z. B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, $\gamma$-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z. B. Buthioninsulfoximine, Homocysteinsulfoximin, Buthioninsulfone, Penta-, Hexa-, Heptathioninsulfoximin) in sehr geringen verträglichen Dosierungen (z. B. pmol bis $\mu$mol/kg), ferner (Metall)-Chelatoren (z. B. $\alpha$-Hydroxyfettsäuren, Palmitinsäure, Phytinsäure, Lactoferrin), $\alpha$-Hydroxysäuren (z. B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z. B. $\gamma$-Linolensäure, Linolsäure, Ölsäure), Folsäure und deren Derivate, Furfurylidensorbitol und dessen Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, $\alpha$-Glycosylrutin, Ferulasäure, Furfurylidenglucitol, Camosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, $ZnSO_4$) Selen und dessen Derivate (z. B. Selenmethionin), Stilbene und deren Derivate (z. B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Zubereitungen gemäß der vorliegenden Erfindung können auch Verwendung als Grundlage für kosmetische oder dermatologische Desodorantien bzw. Antitranspirantien finden. Alle für Desodorantien bzw. Antitranspirantien gängigen Wirkstoffe können vorteilhaft genutzt werden, beispielsweise Geruchsüberdecker wie die gängigen Parfümbestandteile, Geruchsabsorber, beispielsweise die in der Patentoffenlegungsschrift DE-P 40 09 347 beschriebenen Schichtsilikate, von diesen insbesondere Montmorillonit, Kaolinit, Ilit, Beidellit, Nontronit, Saponit, Hectorit, Bentonit, Smectit, ferner beispielsweise Zinksalze der Ricinolsäure.

Keimhemmende Mittel sind ebenfalls geeignet, in die erfindungsgemäßen Zubereitungen eingearbeitet zu werden. Vorteilhafte Substanzen sind zum Beispiel 2,4,4'-Trichlor-2'-hydroxydiphenylether (Irgasan), 1,6-Di-(4-chlorphenylbiguanido)-hexan (Chlorhexidin). 3.4,4'-Trichlorcarbanilid, quaternäre Ammoniumverbindungen, Nelkenöl, Minzöl, Thymianöl, Triethylcitrat, Farnesol (3,7,11-Trimethyl-2,6,10-dodecatrien-1-ol) sowie die in den Patentoffenlegungsschriften DE-37 40 186, DE-39 38 140, DE-42 04 321, DE-42 29 707, DE-43 09 372, DE-44 11 664, DE-195 41 967, DE-195 43 695, DE-195 43 696, DE-195 47 160, DE-196 02 108, DE-196 02 110, DE-196 02 111, DE-196 31 003, DE-196 31 004 und DE-196 34 019 und den Patentschriften DE-42 29 737, DE-42 37 081, DE-43 24 219, DE-44 29 467, DE-44 23 410 und DE-195 16 705 beschriebenen Wirkstoffe bzw. Wirkstoffkombinationen. Auch Natriumhydrogencarbonat ist vorteilhaft zu verwenden.

Die Menge solcher Wirkstoffe (eine oder mehrere Verbindungen) in den Zubereitungen gemäß der Erfindung beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Die Wasserphase der kosmetischen Zubereitungen im Sinne der vorliegenden Erfindung kann auch Gelcharakter aufweisen, die neben einem wirksamen Gehalt am erfindungsgemäß eingesetzten Substanzen und dafür üblicherweise verwendeten Lösungsmitteln, bevorzugt Wasser, noch weitere organische Verdickungsmittel, z.B. Gummiarabikum, Xanthangummi, Natriumalginat, Stärke und Stärkederivate (z. B. Distärkephosphat), Cellulose, Cellulose-Derivate, vorzugsweise Methylcellulose, Hydroxymethylcellulose, Hydroxyethylcellulose, Hydroxypropylcellulose, Hydroxypropylmethylcellulose oder anorganische Verdickungsmittel, z. B. Aluminiumsilikate wie beispielsweise organisch modifizierte oder auch unmodifizierte Hectorite, Bentonite, oder dergleichen, oder ein Gemisch aus Polyethylenglykol und Polyethylenglykolstearat oder -distearat, enthalten. Das Verdickungsmittel ist in dem Gel z. B. in einer Menge

zwischen 0,1 und 30 Gew.-%, bevorzugt zwischen 0,5 und 15 Gew.-%, enthalten.

Ferner kann es von Vorteil sein, Zubereitungen gemäß der Erfindung grenz- bzw. oberflächenaktive Agentien zuzufügen, beispielsweise kationische Emulgatoren wie insbesondere quaternäre Tenside.

Quaternäre Tenside enthalten mindestens ein N-Atom, das mit 4 Alkyl- oder Arylgruppen kovalent verbunden ist. Dies führt, unabhängig vom pH Wert, zu einer positiven Ladung. Vorteilhaft sind Alkylbetain, Alkylamidopropylbetain und Alkyl-amidopropylhydroxysulfain. Die erfindungsgemäß verwendeten kationischen Tenside können ferner bevorzugt gewählt werden aus der Gruppe der quaternären Ammoniumverbindungen, insbesondere Benzyltrialkylammoniumchloride oder -bromide, wie beispielsweise Benzyldimethylstearylammoniumchlorid, ferner Alkyltrialkylammoniumsalze, beispielsweise beispielsweise Cetyltrimethylammoniumchlorid oder -bromid, Alkyldimethylhydroxyethylammoniumchloride oder -bromide, Dialkyldimethylammoniumchloride oder -bromide, Alkylamidethyltrimethylammoniumethersulfate, Alkylpyridiniumsalze, beispielsweise Lauryl-oder Cetylpyrimidiniumchlorid, Imidazolinderivate und Verbindungen mit kationischem Charakter wie Aminoxide, beispielsweise Alkyldimethylaminoxide oder Alkylaminoethyldimethylaminoxide. Vorteilhaft sind insbesondere Cetyltrimethylammoniumsalze zu verwenden.

Vorteilhaft ist auch, kationische Polymere (z. B. Jaguar® C 162 [Hydroxypropyl Guar Hydroxypropyltrimonium Chloride] bzw. modifizierten Magnesiumaluminiumsilikaten (z. B. Quaternium-18-Hectorit, welches z. B. unter der Handelsbezeichnung Bentone® 38 bei der Firma Rheox erhältlich ist, oder Stearalkonium Hectorit, welches z. B. unter der Handelsbezeichnung Softisan® Gel bei der Hüls AG erhältlich ist), einzusetzen.

Erfindungsgemäße Zubereitungen können vorteilhaft auch Ölverdickungsmittel enthalten, um die taktilen Eigenschaften der Emulsion und die Stiftkonsistenz zu verbessern. Vorteilhafte Ölverdickungsmittel im Sinne der vorliegenden Erfindung sind beispielsweise weitere Feststoffe, wie z. B. hydrophobe Siliciumoxide des Typs Aerosil®, welche von der Degussa AG erhältlich sind. Vorteilhafte Aerosil®-Typen sind beispielsweise Aerosil® OX50, Aerosil® 130, Aerosil® 150, Aerosil® 200, Aerosil® 300, Aerosil® 380, Aerosil® MOX 80, Aerosil® MOX 170, Aerosil® COK 84, Aerosil® R 202, Aerosil® R 805, Aerosil® R 812, Aerosil® R 972, Aerosil® R 974 und/oder Aerosil® R976.

Ferner sind auch sogenannte Metallseifen (d. h. die Salze höherer Fettsäuren mit Ausnahme der Alkalisalze) vorteilhafte Ölverdickungsmittel im Sinne der vorliegenden Erfindung, wie beispielsweise Aluminium-Stearat, Zink-Stearat und/oder Magnesium-Stearat. Ebenfalls vorteilhaft ist, Zubereitungen gemäß der Erfindung amphotere bzw. zwitterionische Tenside (z. B. Cocoamidopropylbetain) und Moisturizer (z. B. Betain) zuzusetzen. Vorteilhaft zu verwendende amphotere Tenside sind beispielsweise Acyl-/dialkylethylendiamin, beispielsweise Natriumacylamphoacetat, Dinatriumacylamphodipropionat, Dinatriumalkylamphodiacetat, Natriumacylamphohydroxypropylsulfonat, Dinatriumacylamphodiacetat und Natriumacylamphopropionat, N-Alkylaminosäuren, beispielsweise Aminopropylalkylglutamid, Alkylaminopropionsäure, Natriumalkylimidodipropionat und Lauroamphocarboxyglycinat.

Die Menge der ober- bzw. grenzflächenaktiven Substanzen (eine oder mehrere Verbindungen) in den Zubereitungen gemäß der Erfindung beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Erfindungsgemäße Zubereitungen können auch Wirkstoffe (eine oder mehrere Verbindungen) enthalten, welche gewählt werden aus der Gruppe: Acetylsalicylsäure, Atropin, Azulen, Hydrocortison und dessen Derivaten, z. B. Hydrocortison-17-valerat, Vitamine, z. B. Ascorbinsäure und deren Derivate, Vitamine der B- und D-Reihe, sehr günstig das Vitamin $B_1$, das Vitamin $B_{12}$ das Vitamin $D_1$, aber auch Bisabolol, ungesättigte Fettsäuren, namentlich die essentiellen Fettsäuren (oft auch Vitamin F genannt), insbesondere die $\gamma$-Linolensäure, Ölsäure, Eicosapentaensäure, Docosahexaensäure und deren Derivate, Chloramphenicol, Coffein, Prostaglandine, Thymol, Campher, Extrakte oder andere Produkte pflanzlicher und tierischer Herkunft, z.B. Nachtkerzenöl, Borretschöl oder Johannisbeerkernöl, Fischöle, Lebertran aber auch Ceramide und ceramidähnliche Verbindungen und so weiter. Vorteilhaft ist es auch, die Wirkstoffe aus der Gruppe der rückfettenden Substanzen zu wählen, beispielsweise Purcellinöl, Eucerit® und Neocerit®.

Die Menge solcher Wirkstoffe (eine oder mehrere Verbindungen) in den Zubereitungen gemäß der Erfindung beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Die nachfolgenden Beispiele sollen die vorliegende Erfindung verdeutlichen.

**Beispiel 1**

| | Gew.-% |
|---|---|
| Stearinsäure/Palmitinsäure | 2,00 |
| PEG-40-Stearat | 2,00 |
| Glycerylstearat | 2,00 |
| Cetylstearylalkohol | 3,00 |

Tabelle fortgesetzt

| | Gew.-% |
|---|---|
| Hydriertes Polyisobuten | 7,00 |
| Petrolatum | 4,00 |
| Cyclomethicon | 9,00 |
| Glyceryllanolat | 2,00 |
| Glycerin | 4,00 |
| Parfüm, Konservierungsmittel, Farbstoffe, Antioxidantien etc. | q.s. |
| Wasser | ad 100,00 |
| pH-Wert eingestellt auf | 6,5 |

**Beispiel 2**

| | Gew.-% |
|---|---|
| Stearinsäure/Palmitinsäure | 1,20 |
| PEG-40-Stearat | 2,00 |
| Glycerylstearat | 4,00 |
| Cetylstearylalkohol | 3,00 |
| Hydriertes Polyisobuten | 10,00 |
| Cyclomethicon | 10,00 |
| Glyceryllanolat | 2,00 |
| Glycerin | 4,00 |
| Parfüm, Konservierungsmittel, Farbstoffe, Antioxidantien etc. | q.s. |
| Wasser | ad 100,00 |
| pH-Wert eingestellt auf | 6,5 |

**Beispiel 3**

| | Gew.-% |
|---|---|
| Stearinsäure/Palmitinsäure | 1,20 |
| PEG-40-Stearat | 2,00 |
| Glycerylstearat | 6,00 |
| Cetylstearylalkohol | 2,50 |
| Hydriertes Polyisobuten | 8,00 |
| Petrolatum | 2,00 |
| Cyclomethicon | 9,00 |
| Glyceryllanolat | 2,00 |
| Glycerin | 4,00 |
| Parfüm, Konservierungsmittel, Farbstoffe, Antioxidantien etc. | q.s. |
| Wasser | ad 100,00 |
| pH-Wert eingestellt auf | 6,5 |

**Beispiel 4**

| | Gew.-% |
|---|---|
| Stearinsäure/Palmitinsäure | 1,20 |

Tabelle fortgesetzt

| | Gew.-% |
|---|---|
| PEG-100-Stearat | 1,20 |
| Glycerylstearat | 3,60 |
| Cetylstearylalkohol | 3,00 |
| Hydriertes Polyisobuten | 9,00 |
| Petrolatum | 2,00 |
| Cyclomethicon | 7,00 |
| Dimethicon | 3,00 |
| Glyceryllanolat | 2,00 |
| Glycerin | 4,00 |
| Parfüm, Konservierungsmittel, Farbstoffe, Antioxidantien etc. | q.s. |
| Wasser | ad 100,00 |
| pH-Wert eingestellt auf | 7,0 |

**Beispiel 5**

| | Gew.-% |
|---|---|
| Stearinsäure/Palmitinsäure | 1,20 |
| PEG-20-Stearat | 1,20 |
| Glycerylstearat | 3,60 |
| Cetylstearylalkohol | 3,00 |
| Hydriertes Polyisobuten | 9,00 |
| Petrolatum | 2,00 |
| Cyclomethicon | 5,00 |
| Dimethicon | 5,00 |
| Glyceryllanolat | 5,00 |
| Glycerin | 10,00 |
| Parfüm, Konservierungsmittel, Farbstoffe, Antioxidantien etc. | q.s. |
| Wasser | ad 100,00 |
| pH-Wert eingestellt auf | 6,5 |

**Beispiel 6**

| | Gew.-% |
|---|---|
| Stearinsäure/Palmitinsäure | 1,20 |
| PEG-40-Stearat | 1,20 |
| Glyceryl Stearate | 3,60 |
| Cetylstearylalkohol | 3,00 |
| Hydriertes Polyisobuten | 8,00 |
| Paraffinum Liquidum | 5,00 |
| Petrolatum | 2,00 |
| Cyclomethicon | 10,00 |
| Glyceryllanolat | 2,00 |
| Glycerin | 10,00 |
| Parfüm, Konservierungsmittel, Farbstoffe, Antioxidantien etc. | q.s. |
| Wasser | ad 100,00 |
| pH-Wert eingestellt auf | 7,0 |

**Beispiel 7**

| | Gew.-% |
|---|---|
| Stearinsäure/Palmitinsäure | 1,20 |
| PEG-40-Stearat | 1,20 |
| Glyceryl-Stearat | 3,60 |
| Cetylstearylalkohol | 3,00 |
| Hydriertes Polyisobuten | 8,00 |
| Polydecene | 6,00 |
| Petrolatum | 4,00 |
| Cyclomethicon | 10,00 |
| Glyceryllanolat | 2,00 |
| Glycerin | 5,00 |
| Parfüm, Konservierungsmittel, Farbstoffe, Antioxidantien etc. | q.s. |
| Wasser | ad 100,00 |
| pH-Wert eingestellt auf | 7,0 |

**Beispiel 8**

| | Gew.-% |
|---|---|
| Stearinsäure/Palmitinsäure | 1,20 |
| PEG-40-Stearat | 2,00 |
| Glycerylstearat | 4,00 |
| Cetearylalkohol | 3,00 |
| Hydriertes Polyisobuten | 9,00 |
| Polydecen | 5,00 |
| Caprylsäure/Caprinsäuretriglyceride | 5,00 |
| Petrolatum | 1,00 |
| Cyclomethicon | 4,00 |
| Glyceryllanolat | 2,00 |
| Glycerin | 3,00 |
| Parfüm, Konservierungsmittel, Farbstoffe, Antioxidantien etc. | q.s. |
| Wasser | ad 100,00 |
| pH-Wert eingestellt auf | 6,0 |

**Beispiel 9**

| | Gew.-% |
|---|---|
| Stearinsäure/Palmitinsäure | 1,20 |
| PEG-40-Stearat | 2,00 |
| Glycerylstearat | 4,00 |
| Cetylstearylalkohol | 3,00 |
| Hydriertes Polyisobuten | 12,00 |
| Polydecen | 4,00 |
| Cyclomethicon | 5,00 |
| Glyceryllanolat | 2,00 |
| Glycerin | 3,00 |

Tabelle fortgesetzt

| | Gew.-% |
|---|---|
| Octylmethoxycinnamat | 3,00 |
| Benzophenon-3 | 2,00 |
| Octylsalicylat | 1,00 |
| Parfüm, Konservierungsmittel, NaOH, Farbstoffe, Antioxidantien etc. | q.s. |
| Wasser | ad 100,00 |
| pH-Wert eingestellt auf | 7,0 |

**Beispiel 10**

| | Gew.-% |
|---|---|
| Stearinsäure/Palmitinsäure | 1,00 |
| PEG-100-Stearat | 2,00 |
| Glycerylstearat | 4,00 |
| Cetylstearylalkohol | 3,00 |
| Hydriertes Polyisobuten | 9,00 |
| Octyldodecanol | 3,50 |
| Dimethicon | 10,00 |
| Myristylmyristat | 4,00 |
| Glycerin | 3,00 |
| Parfüm, Konservierungsmittel, Farbstoffe, Antioxidantien etc. | q.s. |
| Wasser | ad 100,00 |
| pH-Wert eingestellt auf | 7,0 |

**Beispiel 11**

| | Gew.-% |
|---|---|
| Stearinsäure/Palmitinsäure | 1,00 |
| PEG-100-Stearat | 2,00 |
| Glycerylstearat | 4,00 |
| Cetylstearylalkohol | 3,00 |
| Hydriertes Polyisobuten | 0,50 |
| Dimethicon | 5,00 |
| Cyclomethicon | 15,00 |
| Myristylmyristat | 4,00 |
| Glycerin | 3,00 |
| Parfüm, Konservierungsmittel, Farbstoffe, Antioxidantien etc. | q.s. |
| Wasser | ad 100,00 |
| pH-Wert eingestellt auf | 7,0 |

**Patentansprüche**

**1.** Kosmetische und dermatologische Zubereitungen in Form von O/W-Emulsionen, enthaltend

(1) 0,1 bis zu 5 Gew.-%, bezogen auf das Gesamtgewicht der Zubereitungen, einer oder mehrerer $C_{14}$-$C_{22}$-Fettsäuren,
(2) 0,2 bis zu 10 Gew.-%, bezogen auf das Gesamtgewicht der Zubereitungen, eines oder mehrerer Mono-

und/oder Diglyceride von Fettsäuren,
(3) 0,1 bis zu 5 Gew.-%, bezogen auf das Gesamtgewicht der Zubereitungen, eines oder mehrerer ethoxylierter Fettsäureester,
wobei die Summe aus (1), (2) und (3) maximal 12 Gew. % beträgt,
(4) 0,5 bis 10 Gew.-%, bezogen auf das Gesamtgewicht der Zubereitungen, eines oder mehrerer unpolarer Lipide,
(5) 0,5 - 7,5 Gew.-%, bezogen auf das Gesamtgewicht der Zubereitungen, eines oder mehrerer Fettalkohole
(6) 0,5 - 7,5 Gew.-%, bezogen auf das Gesamtgewicht der Zubereitungen, eines oder mehrerer lipophiler Konsistenzgeber eines Schmelz- oder Tropfpunktes ≥ 30 °C
(7) 0,5 - 10 Gew.-%, bezogen auf das Gesamtgewicht der Zubereitungen, an einem oder mehreren Siliconölen,
(8) wobei die gesamte Lipidphase bis zu 40 Gew.-% polare Lipide enthalten kann, bezogen auf das Gesamtgewicht der Lipidphase.

**2.** Kosmetische und dermatologische Zubereitungen nach Anspruch 1, enthaltend

(1) 0,5 bis zu 1,0 Gew.-%, bezogen auf das Gesamtgewicht der Zubereitungen, einer oder mehrerer $C_{14}$-$C_{22}$-Fettsäuren.

**3.** Kosmetische und dermatologische Zubereitungen nach Anspruch 1, enthaltend

(2) 2,5 bis zu 3,0 Gew.-%, bezogen auf das Gesamtgewicht der Zubereitungen, eines oder mehrerer Mono- und/oder Diglyceride von Fettsäuren.

**4.** Kosmetische und dermatologische Zubereitungen nach Anspruch 1, enthaltend

(3) 1,0 bis zu 1,5 Gew.-%, bezogen auf das Gesamtgewicht der Zubereitungen, eines oder mehrerer ethoxylierter Fettsäureester.

**5.** Kosmetische und dermatologische Zubereitungen nach Anspruch 1, enthaltend

(2) bis zu 10 Gew.-%, bezogen auf das Gesamtgewicht der Zubereitungen, an Glycerylstearat.

**6.** Kosmetische und dermatologische Zubereitungen nach Anspruch 1, enthaltend

(3) bis zu 5 Gew.-%, bezogen auf das Gesamtgewicht der Zubereitungen, eines oder mehrerer ethoxylierter Fettsäureester gewählt aus der Gruppe der PEG-20- bis PEG-100-Stearate.

**7.** Kosmetische und dermatologische Zubereitungen nach Anspruch 1, enthaltend

(4) 0,5 bis 10 Gew.-%, bezogen auf das Gesamtgewicht der Zubereitungen, eines oder mehrerer unpolarer Lipide, gewählt aus der Gruppe Mineralöl und/oder Mineralwachse (u.a. Vaseline)

**8.** Kosmetische und dermatologische Zubereitungen nach Anspruch 1, enthaltend

(5) 2,0 - 3,0 Gew.-%, bezogen auf das Gesamtgewicht der Zubereitungen, eines oder mehrerer Fettalkohole.

**9.** Kosmetische und dermatologische Zubereitungen nach Anspruch 1, enthaltend

(6) 1,5 - 2,5 Gew.-%, bezogen auf das Gesamtgewicht der Zubereitungen, eines oder mehrerer lipophiler Konsistenzgeber eines Schmelz- oder Tropfpunktes ≥ 30 °C

**10.** Kosmetische und dermatologische Zubereitungen nach Anspruch 1, enthaltend

(7) 5 - 8 Gew.-%, bezogen auf das Gesamtgewicht der Zubereitungen, an einem oder mehreren Siliconölen, besonders bevorzugt Cylomethicone

## Claims

1. Cosmetic and dermatological preparations in the form of O/W emulsions, comprising

(1) 0.1 up to 5% by weight, based on the total weight of the preparations, of one or more $C_{14}$-$C_{22}$-fatty acids,
(2) 0.2 up to 10% by weight, based on the total weight of the preparations, of one or more mono- and/or diglycerides of fatty acids,
(3) 0.1 up to 5% by weight, based on the total weight of the preparations, of one or more ethoxylated fatty acid esters,
where the sum of (1), (2) and (3) is at most 12% by weight,
(4) 0.5 to 10% by weight, based on the total weight of the preparations, of one or more nonpolar lipids,
(5) 0.5 - 7.5% by weight, based on the total weight of the preparations, of one or more fatty alcohols
(6) 0.5 - 7.5% by weight, based on the total weight of the preparations, of one or more lipophilic bodying agents having a melting point or dropping point of ≥ 30°C,
(7) 0.5 - 10% by weight, based on the total weight of the preparations, of one or more silicone oils,
(8) where the total lipid phase can comprise up to 40% by weight of polar lipids, based on the total weight of the lipid phase.

2. Cosmetic and dermatological preparations according to Claim 1, comprising

(1) 0.5 up to 1.0% by weight, based on the total weight of the preparations, of one or more $C_{14}$-$C_{22}$-fatty acids.

3. Cosmetic and dermatological preparations according to Claim 1, comprising

(2) 2.5 up to 3.0% by weight, based on the total weight of the preparations, of one or more mono- and/or diglycerides of fatty acids.

4. Cosmetic and dermatological preparations according to Claim 1, comprising

(3) 1.0 up to 1.5% by weight, based on the total weight of the preparations, of one or more ethoxylated fatty acid esters.

5. Cosmetic and dermatological preparations according to Claim 1, comprising

(2) up to 10% by weight, based on the total weight of the preparations, of glyceryl stearate.

6. Cosmetic and dermatological preparations according to Claim 1, comprising

(3) up to 5% by weight, based on the total weight of the preparations, of one or more ethoxylated fatty acid esters chosen from the group of PEG-20 to PEG-100 stearates.

7. Cosmetic or dermatological preparations according to Claim 1, comprising

(4) 0.5 to 10% by weight, based on the total weight of the preparations, of one or more nonpolar lipids, chosen from the group of mineral oil and/or mineral waxes (including Vaseline).

8. Cosmetic and dermatological preparations according to Claim 1, comprising

(5) 2.0 - 3.0% by weight, based on the total weight of the preparations, of one or more fatty alcohols.

9. Cosmetic and dermatological preparations according to Claim 1, comprising

(6) 1.5 - 2.5% by weight, based on the total weight of the preparations, of one or more lipophilic bodying agents having a melting point or dropping point of ≥ 30°C.

10. Cosmetic and dermatological preparations according to Claim 1, comprising

(7) 5 - 8% by weight, based on the total weight of the preparations, of one or more silicone oils, particularly

preferably cyclomethicones.

## Revendications

1. Préparations cosmétiques et dermatologiques sous forme d'émulsions H/E, contenant

(1) 0,1 à 5 % en poids, par rapport au poids total des préparations, d'un ou plusieurs acides gras en $C_{14}$-$C_{22}$,
(2) 0,2 à 10 % en poids, par rapport au poids total des préparations, d'un ou plusieurs mono- et/ou diglycérides d'acides gras,
(3) 0,1 à 5 % en poids, par rapport au poids total des préparations, d'un ou plusieurs esters d'acides gras éthoxylés, la somme de (1), (2) et (3) étant au maximum de 12 % en poids,
(4) 0,5 à 10 % en poids, par rapport au poids total des préparations, d'un ou plusieurs lipides non polaires,
(5) 0,5 - 7,5 % en poids, par rapport au poids total des préparations, d'un ou plusieurs alcools gras,
(6) 0,5 - 7,5 % en poids, par rapport au poids total des préparations, d'un ou plusieurs agents de consistance lipophiles ayant un point de fusion ou de goutte ≥ 30°C,
(7) 0,5 - 10 % en poids, par rapport au poids total des préparations, d'une ou plusieures huiles de silicone,
(8) la phase lipidique totale pouvant contenir jusqu'à 40 % en poids de lipides polaires, par rapport au poids total de la phase lipidique.

2. Préparations cosmétiques et dermatologiques selon la revendication 1, contenant

(1) 0,5 à 1,0 % en poids, par rapport au poids total des préparations, d'un ou plusieurs acides gras en $C_{14}$-$C_{22}$.

3. Préparations cosmétiques et dermatologiques selon la revendication 1, contenant

(2) 2,5 à 3,0 % en poids, par rapport au poids total des préparations, d'un ou plusieurs mono- et/ou diglycérides d'acides gras.

4. Préparations cosmétiques et dermatologiques selon la revendication 1, contenant

(3) 1,0 à 1,5 % en poids, par rapport au poids total des préparations, d'un ou plusieurs esters d'acides gras éthoxylés.

5. Préparations cosmétiques et dermatologiques selon la revendication 1, contenant

(2) jusqu'à 10 % en poids, par rapport au poids total des préparations, de stéarate de glycéryle.

6. Préparations cosmétiques et dermatologiques selon la revendication 1, contenant

(3) jusqu'à 5 % en poids, par rapport au poids total des préparations, d'un ou plusieurs esters d'acides gras éthoxylés choisis dans le groupe des stéarates de PEG-20 à PEG-100.

7. Préparations cosmétiques et dermatologiques selon la revendication 1, contenant

(4) 0,5 - 10 % en poids, par rapport au poids total des préparations, d'un ou plusieurs lipides non polaires, choisis dans le groupe constitué par l'huile minérale et/ou des cires minérales (la vaseline en particulier).

8. Préparations cosmétiques et dermatologiques selon la revendication 1, contenant

(5) 2,0 - 3,0 % en poids, par rapport au poids total des préparations, d'un ou plusieurs alcools gras.

9. Préparations cosmétiques et dermatologiques selon la revendication 1, contenant

(6) 1,5 - 2,5 % en poids, par rapport au poids total des préparations, d'un ou plusieurs agents de consistance lipophiles ayant un point de fusion ou de goutte ≥ 30°C.

10. Préparations cosmétiques et dermatologiques selon la revendication 1, contenant

(7) 5 - 8% en poids, par rapport au poids total des préparations, d'une ou plusieures huiles de silicone, particulièrement préférablement les cyclomethicones.